# EUROPEAN PATENT SPECIFICATION

(11) **EP 2 615 059 B1**
(45) Date of publication and mention of the grant of the patent: **08.11.2017**
(21) Application number: 11823572.0
(22) Date of filing: 06.09.2011
(51) Int. Cl.: C01B 17/64, C01G 5/00, C07C 229/08, C07F 1/10, G01N 21/65

(54) **METHOD FOR PRODUCING METAL COMPLEX QUANTUM CRYSTALS**
VERFAHREN ZUR HERSTELLUNG VON METALLKOMPLEX-QUANTUMKRISTALLEN
PROCÉDÉ DE PRODUCTION DE CRISTAUX QUANTIQUES DE COMPLEXES MÉTALLIQUES

(30) Priority: 06.09.2010 JP 2010198555
(43) Date of publication of application: 17.07.2013
(73) Proprietor: MYTECH Co., Ltd., Himeji-shi, Hyogo 679-2101 (JP)
(72) Inventor: HASEGAWA, Yuki, Himeji-shi Hyogo 679-2101 (JP); HASEGAWA, Katsuyuki, Himeji-shi Hyogo 679-2101 (JP)
(74) Representative: Sielewiesiuk, Jakub
(86) International application number: PCT/JP2011/070274
(87) International publication number: WO 2012/033097

(56) References cited:
- EP-A1- 2 405 257
- WO-A1-2008/010442
- WO-A1-2009/075471
- WO-A2-2006/065762
- JP-A- 7 146 295
- JP-A- 2006 083 450
- JP-A- 2007 198 933
- JP-A- 2008 281 529
- JP-A- 2010 203 973
- US-A1- 2005 191 665
- US-A1- 2008 166 706

## Description

### Technical Field

The present invention relates to a method of producing a quantum crystal of metal complex including a metal nano cluster or nano clusters as metal quantum dots by using an aqueous solution of the metal complex and its use based on the effect of locarized surface plasmon enhancement.

### Background

As a typical material of the next generation in the nano technology, surface modified nano particles, which are made from metal atoms by a technology for control in a range of nano level form and a nano size so as to form a nano cluster or nano clusters, are getting much attention, because nano clusters can be designed to have a new electronic property or materiality due to so-called quantum size effect which would happen in nanometer area. Hereinafter, "nano cluster" means agglomeration of several to several hundred atoms and molecules having a size of several nm to several 10 nm. It is said that these clusters are bigger than a single molecule, and smaller than nano crystal, so that nano cluster is a material which shows a unique function or property different from that in a state of atoms, molecules and bulk crystals. Therefore, by controlling the size and the number of material composition atom, it is expected a new knowledge and a finding concerning a phase transition, a crystal growth, a chemical reaction, catalytic action, and so on. One of them is a so-called Surface Plasmon Resonance (SPR) on the surface of the metal. Although it is general that an electron in the metal does not tend to interact with a light, the light can interact with electrons existing in metal nano particles under a special condition, resulting in occurrence of so called Localized Surface Plasmon Resonance. Especially, according to the theoretical consideration of so-called "dimmer of nano Ag particles" in case that the silver nano particles are positioned in a predetermined distance, it is thought that the degree of electric field enhancement made by the wavelength around 400nm would be especially very high, and in case of less than 400nm, there would exist a peak at the wavelength around 300 nm. And, in relations with the particles diameter, it is thought that, as a particle diameter is widened, the position of the peak becomes high , further the peak would shift to the long wave side and the peak width would become bigger, so that the field enhancement effect can be expected in a wide range of wavelength..

WO 2006/065762 discloses a method for enhancing a Raman signal including the step of providing a seed comprising a gold nanoparticle; providing an aqueous growth solution comprising cethyltrimetylammonium bromide, hydrogen tetrachloroaurate, and ascorbic acid; and adding a quantity of the seed solution to the aqueous growth solution. The method described is a chemical deposition process, and the document is completely silent on any aspects that might relate to galvanic deposition due to electro-potential difference between the metal complex and the metal carrier.

Further, US 2008/166706 A1 discloses a chemical sensor comprising a plurality of particles, each particle comprising a core, a shell having at least one surface and having contact with the core and wherein the shell comprises a sulfur oxygen molecular species, and wherein the particle has been selectively sized using a notch filter and electromagnetic radiation, the electromagnetic radiation having a spectral wavelength of between 350 nm and about 1075 nm. The method described is also a chemical deposition process, as it includes a step of combing HAuCl4 with Na2S, followed by incubating the combined solution.

Therefore, according to the theory of so-called dimmer of nano Ag particles it has been proposed a lot of measuring substrates for SERS (Surface Enhance Raman Scattering), wherein in order to accumulate nano particles of Noble metal such as Ag, Au and so on having a diameter of about several 10 nm, there have been used a Chemical Vapor Deposition (CVD) method on a glass substrate and a coloidal particle deposition method of Ag or Au in an aqueous solution on a chemical decolated such as legin or cyan decolated glass substrate (Refer to the below Non-patent literatures 1 to 3 and patent document 1). Therefore, at the present the above CVD method has now to be used for mass-production of the SERS substrate.

### [Prior Art literature]

### [Patent documents]

[Patent documents 1] Tokkukaihei 11 (1999)-61209

### [Non-patent literature]

[Non-patent literature 1]
   S. Nie and S.R.Emory,Science.275,1102 (1997)
[Non-patent literature2]
   K.C.Grabar,P.C.Smith,M.D.Musick,J.A.Davis,D.G.Walter,M.A.Jackson,A.P.Guthr ie and M.J.Natan,J.Am.Chem,Soc.,118,1148 (1996)
[Non-patent literature3]
   R.M. Bright, M.D. Musick and M.H.Natan,Langmuir,14, 5695 (1998)

### [The summary of the invention]

### [The problem to be solved by the invention]

However, the CVD substrate has not a function for absorbing the sample to be detected, so that the substrate for SERS detecting has to be prepared by so-called drop & dry method including coating and drying steps of the sample to be detected, and there is a difficulty in relation to the prompt detection and a facult in the matter of the deterioration of the sample due to a long detecting time. Furthermore, there is the biggest fault in the matter of a poor repeatability of the measurement, resulting in a great problem of barrier for a variety applications of the SERS method. Therefore, the inventors came back to the fundamental points of the SERS function and/or phenomenon and deeply thought it to overcome the above problems. It is said that the degree of enhancement in the surface plasmon phenomenon is depended on a variety of physical and chemical parameters including various atom bonding position and orientation existing in absorbed molecules on the electro-magnetic field of the metal surface. Accordingly, in order to provide a best substrate for keeping a good repeatability in relation of the SERS measuring, it is necessary to develop the substrate from the following two points relating to the SERS mechanism ; (i) achievement of the best physical condition for surface plasmon resonance in the metal to enhance a local strength of the injection light and (ii) achievement of the best chemical condition for formation of so-called charge-transfer complex and electrocharge transition between metal surfaces and Raman active molecules to be absorbed. Thinking about the substrate from the original point of SERS sharply, we have found that it is possible to apply the CVD method as long as the physical condition is concerned, although it is difficult to control the particle size and orientation and at the same time, we have come to find that it is impossible for the CVD method to achieve the best chemical condition for formation of the charge-transfer complex.

By the way, it was reported that Prof. Dr. Kimura of Hyogo prefectural university succeeded in the preparation of a two or three dimensional artificial particle crystal by using nano particles as component or element for the crystal recently, and further succeeded in the IV group cluster crystal from the aqueous solution by using the Si cluster in the world.
The crystal of these particles can be said as the quantum dot crystal which has the periodic structure of nanometer, and it has come to be expected as the key material of the nano-technology in the 21st century. Taking the above findings and knowledge into consideration, in order to provide a new method for making a crystal of metal quantum dots on the substrate in place of the prior CVD method, we have made a lot of research by using a various kinds of metal complex in the aqueous solution and have found that, in case that a plasmon metal can coordinate with a ligand and form a metal complex in the aqueous solution, the metal complex tends to be crystallized in a form of quantum crystals on a metal substrate and metal nano clusters encapsulated in the quantum crystals form quantum dots, resulting in observation of strong plasmon enhancement effect which never been seen before. Maybe it would be caused by a function due to the formation of charge-transfer complex.

### [The means to solve the problem]

The present invention relates to a method of producing a quantum crystal of metal complex, more specifically - an Ag complex having plasmon enhancement effect and has been made on the basis of the above new knowledge, which comprises a step of providing an aqueous solution of the Ag complex consisting of a ligand and Ag, a step of contacting the aqueous solution of the Ag complex with a carrier of a metal or a metal alloy carrier, more specifically - a carrier of Cu or Cu alloy showing an electrode potential lower (ionization tendency higher) than that of Ag or with the Cu or Cu alloy carrier being adjusted at an electrode potential lower than that of Ag in the aqueous solution and a step of depositing the Ag complex from the aqueous solution on the Cu or Cu alloy carrier substrate to form and arrange quantum crystals of the Ag complex on the Cu or Cu alloy carrier, wherein the deposition is taking place due to electro-potential difference between the Ag complex and the Cu or Cu alloy carrier.

It is surprised that the deposition and agglomeration of plasmon metal complex from the aqueous solution on the metal substrate is effective on production of the 1 conditions relating to mechanism of SERS phenomenon, one of which is the achievement of (i)the best physical condition for surface plasmon resonance enhancing the local strength of injection light and (ii) the best chemical condition for formation of the charge-transfer complex between the metal surface and Raman active molecules.

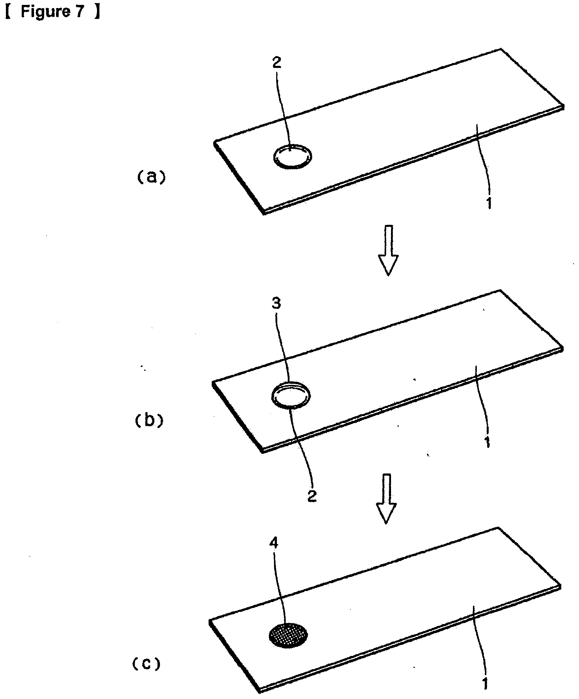

As a useful carrier body, one of the group consisting of a metal substrate, a metal particle, a metal needle, a metal film inside of the capillary may be chosen corresponding to the use of metal quantum crystal. In case of silver plasmon metal, the carrier of metal should be chosen in order to have an electrode potential lower (ionization tendency higher) than that of Ag plasmon metal or complex to be deposited on the metal carrier, for example the carrier metal may be selected from the group consisting of copper, brass and phosphor bronze, and the form of the substrate is adopted so as to receive a drop of the metal complexesolution as shown in Fig.7, on which the aqueous solution of sample to be measured is put a drop on the prepared substrate and then SERS inspection may be done by irradiation of laser. In case of the metal particles provided with quantum crystals of the plasmon metal complex, it can be used for a light transmission electrode material used at the light injection side of the solar battery. In case of a metal needle provided with quantum crystals of the plasmon metal complex, it can be used for the thermal therapy, wherein the metal needle is inserted into the affected part directly, and the quantum crystal part becomes hot due to surface enhancement resonance by the laser irradiation. In case of a capillary including quantum crystals of the plasmon metal complex, the small sample, is absorbed therein without being contaminated. Therefore, the present inventive quantum crystal can be formed on the metal film made by chemical deposition or metal parts inserted in the capillary.

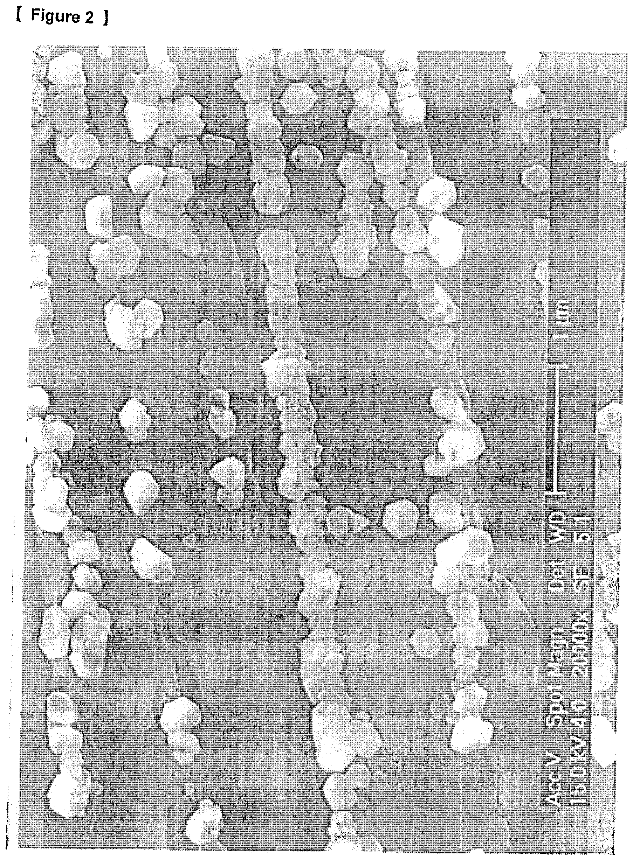

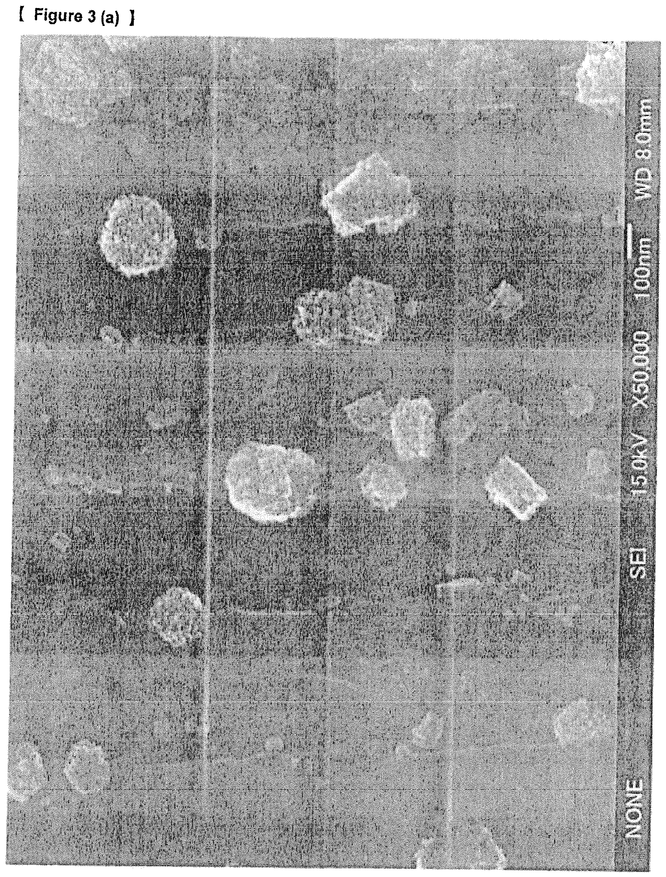

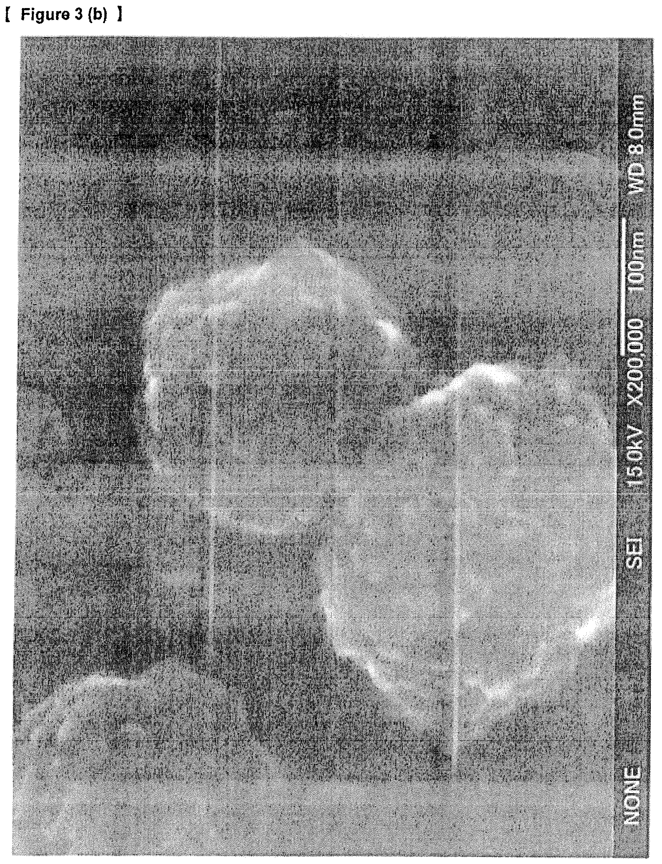

The metal complex is formed from the inventive quantum crystal encapsulating nano cluster of plasmon metal particles on the metal substrate (refer to Fig. 2 and Fig.3(a) and (b)). From the effect of strong plasmon enhancement on the surface of plasmon metal, it can be guessed that the plasmon metal particles are agglomerated in a state of nano clusters having average size of several nm to ten and several nm to make quantum dots having nano size and being arranged at a certain regular intervals, so that the best physical condition can be provided on the substrate, thinking from the aspect of the surface plasmon resonance enhancing local strength of the injection light.

Further, as the metal complex is crystallized on the metal substrate, it can be guessed that at least a part of plasmon metal particles to form nano cluster is deposited in a state of metal and also a remainder of plasmon metal particles is deposited in a state of a metal complex ion bonding with ligand, so that plasmon metal complexes can keep or make an ionized state which can absorb a sample to be detected by drop of the solution and (ii) plasmon metal particles and Raman active molecules make a charge-transfer complexeso that SERS detection can be instantly applied in a state of solution of the sample without drying before detection.

The plasmon metal in the present invention can coordinate with a various kinds of ligands so as to deposit metal complexes from the aqueous solution on the metal substrate. The ligand to be chosen for the metal complexeshould be determined, considering important parameters relating to the formation of the quantum crystal of metal complexes such as a constant of the metal complex stability in the aqueous solution, a complex structure coordinated with ligands and so on.
It is important that at least the selection of ligands should be done, considering that the metal complex to be formed between the plasmon metal and the ligand becomes a complex for forming a charge-transfer complex with the sample. At the present, a ligand selected from the group consisting of amino acid ion, anmmonia ion, a thiosulfate ion and nitrate ion, can form the plasmon metal complex and coordinate with the plasmon metal quantum dots as a ligand, which surface plasmon resonance can be detected by the SERS method. As a result, it is guessed that, (i) the best physical condition for surface plasmon resonance enhancing local strength of injection light and (ii) the best chemical condition for formation of the charge-transfer complex between the metal surface and Raman active molecules and transition thereafter, can be achieved by formation of the quantum crystal of the plasmon metal complexes so that it comes to know that the sample to be detected by SERS method had better be ion-bonded with plasmon metal particles by electro-charge.

When the above plasmon metal complex is formed according to the present invention, it is desirable that one selected from the group consisting of Au (gold), Ag (silver) and Cu (copper) should be chosen as the plasmon metal.

The aqueous solution of plasmon metal complex in the present invention, is used for making the metal complex to be depisited and agglomerated on the metal carrier in order to produce nano metal dots arranged in a suitable intervals on the metal carrier. The concentration of 500 to 2,000 ppm is especially desirable, because in case of less than 500 ppm it is impossible to form nano metal dots in the suitable intervals or it needs a long time to make nano metal dots in the suitable intervals. On the other hand, in case of more than 2,000 ppm it is difficult to control the dots in a suitable intervals due to the prompt deposition and agglomation of the metal complex.
In case of Ag plasmon metal, a determined amount of silver chloride should be added into the aqueous solution including a ligand compound such as ammonia and a sodium thiosulfate to make silver complex with the ligand. The preservation of the metal complex solution can be improved when a dispersant is added in order to prevent the metal complex or its ion from progress of agglomeration in the aqueous solution as long as a measurement is not obstructed.

In case of Ag nano cluster (cluster of 10 -20 nm includes from twenty to forty atoms) the dispersant may be added at 1/50 to 1/150 molar rate as the Ag atomic weight equivalent. It can get a good localized plasmon enhancing effect with a silver thiosulfate complex, a silver anmine complex, a silver nitrate complex and a silver amino acid complex in a range of Ag equivalent of 500 - 2000ppm.

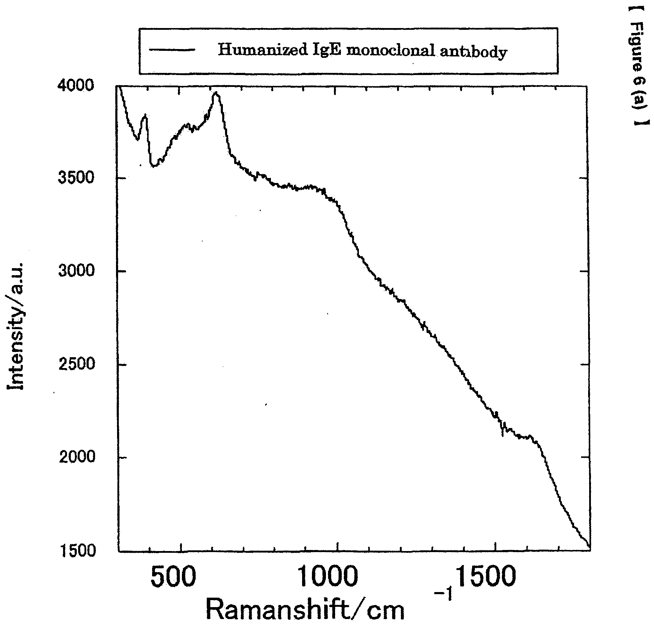

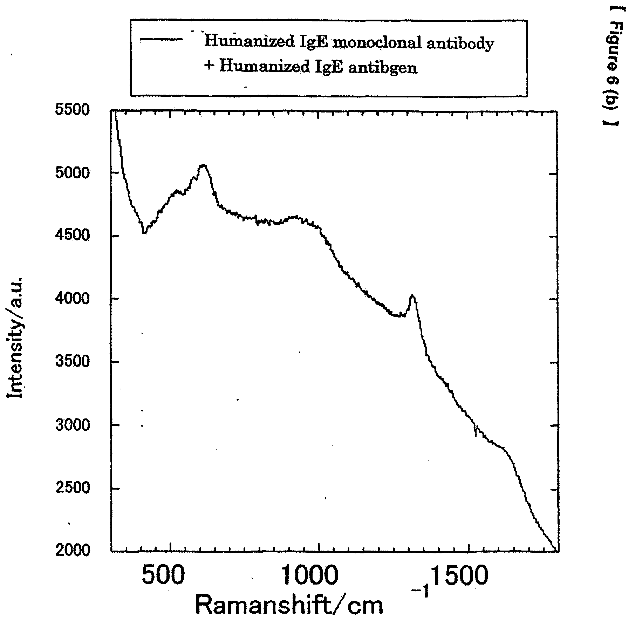

In the present invention, an antibody can be absorbed on the plasmon metal dots through an electro-charge therebetween as a ligand or a substitute of ligand, so that the antigen-antibody reaction can be detected by using this invention method(refer to Figure 6 (a) and (b))

### [The effect of the invention]

1) If the metal complex in the aquous solution can be made of a various kinds of ligands and the plasmon metal to be cordinated with the ligand, the metal complex in the aqueous solution can be reductively deposited on the metal carrier by potential difference between the metal complex and the metal carrier to make the metal complex crystallized so as to encapsulate quantum crystals having a suitable quantum size and formed in a controlled arrangement which is the best physical condition, resulting in achievement of a desirable quantum size effect. Among them, the nano cluster of Au, Ag and Cu tends to form quantum dots which is useful materials for surface plasmon resonance excitation element.
2) Moreover, the metal complex leads to a good result of the repeatability in the SERS detection method, because it may be in the condition that a metal element of the metal complex is easy to be ionized and absorb the Raman activated molecule on the ionized surface of the metal or metal complex to form a charge-transfer complex which is the best chemical condition.
3) The metal complex is easy to control a charge property into a plus or minus which is easy to absorb a protein such as virus and so on. For example, if quantum dots of the metal complex are bonded with a protein Avidin or a living body material Biotin and deposited on the metal substrate, a SERS substrate suitable for the protein detection can be provided.
4) The metal complex is also possible to be bonded with an antibody in the aqueous solution by adding the antibody thereinto and dispersing it in the aqueous solution. Therefore, if the metal complex together with the antibody is deposited on the metal substrate, the choice of the antibody can provide a suitable substrate for absorbing a predetermined antigen and detecting the antigen in the antigen-antibody reaction.
5) If the metal complex is formed in the point of a metal needle, which can be inserted into the affected part directly, the plasmon enhancement effect is obtained by irradiation of the laser light, so that the needle point becomes hot and a hyperthermia therapy can be enforced in the affected part.

It is observed that the plasmon metal complex made of a silver coordinated with thiosulfate ion produces plate-like crystals of 100 to 200 nm on the metal substrate, which shows the excellent effects of surface plasmon resonance exicitation and electro-field reinforcement, which may be probably resulted from the formation of quantum dots made by Ag nano cluster in the hexagonal plate-like crystals (as shown in Fig. 3 (a) and (b)).

### [The simple explanation of the drawings]

[Figure 1]

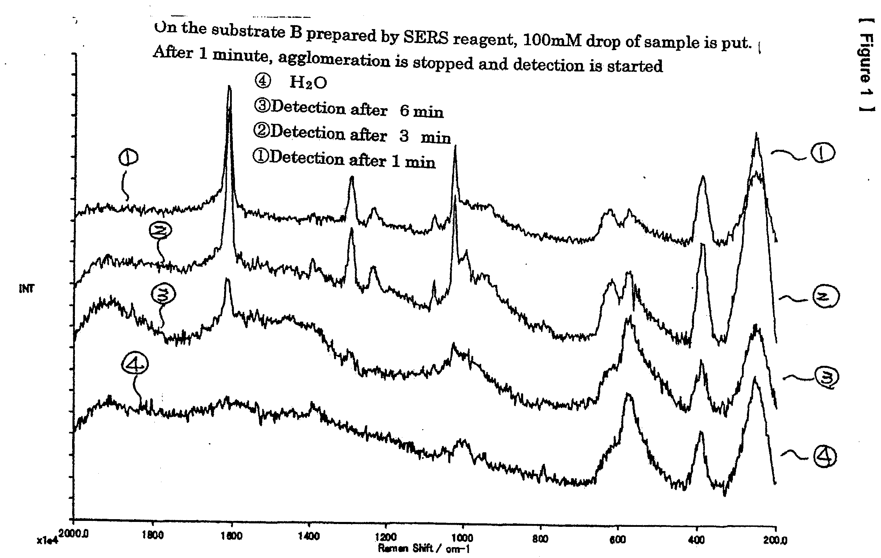

   Fig.1 is a graph showing a Raman scattering spectrum of 4,4'-bipyridine of 100 nM on a SERS substrate prepared according to the present invention method, from which an excellent plasmon resonance excitation effect is observed.
[Figure 2]
   Fig.2 is a photograph (20,000 times) of scanning type electron microscope, which shows a state of formation that the silver complex crystal forms a hexagonal plate-like crystal on the brass substrate.
[Figure 3 (a)]
   Fig.3(a) is a photograph (50,000 times) of scanning type electron microscope, which shows a state of formation that the silver complex crystal forms quantum dots on the phosphor bronze substrate.
[Figure 3 (b)]
   Fig.3(b) is a photograph (200,000 times) of scanning type electron microscope which shows the state that the silver complex crystal forms quantum dots crystal on the phosphor bronze substrate.
[Figure 4 (a)]

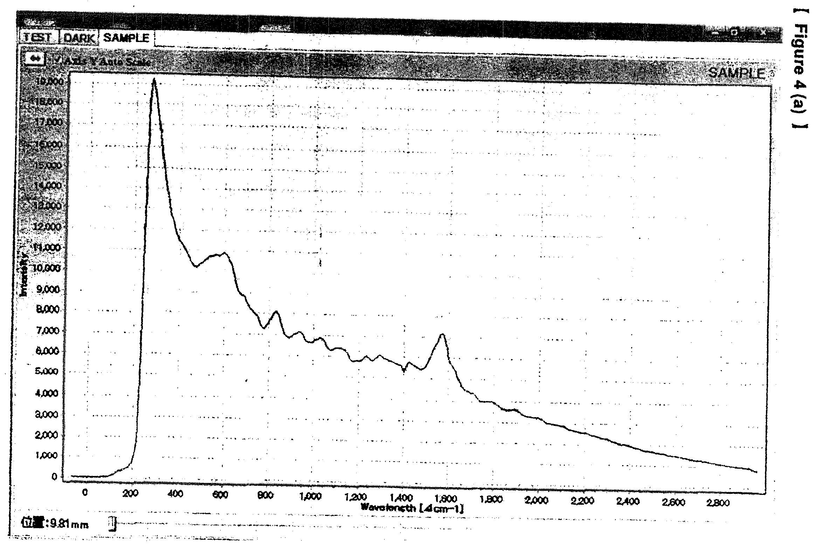

   Fig.4(a) is a graph showing a Raman scattering spectrum of pure water of 100 nM on Ag complex substrate prepared by deposition of Ag nitrate aqueous solution on phosphor bronze according to the presentinvention method.
[Figure 4 (b)]

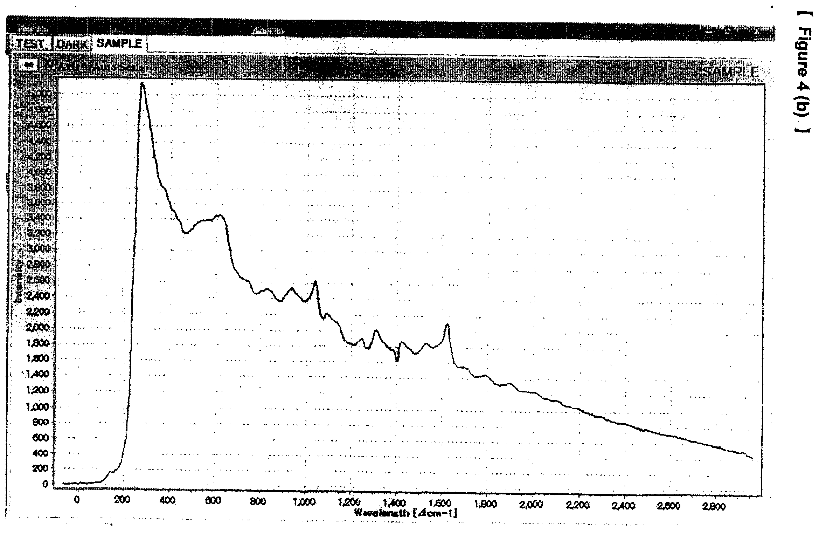

   Fig.4(b) is a graph showing a Raman scattering spectrum of 4,4'-bipyrijin of 100 nM on Ag complex substrate prepared by deposition of Ag nitrate aqueous solution on phosphor bronze according to the present invention method.
[Figure 5]

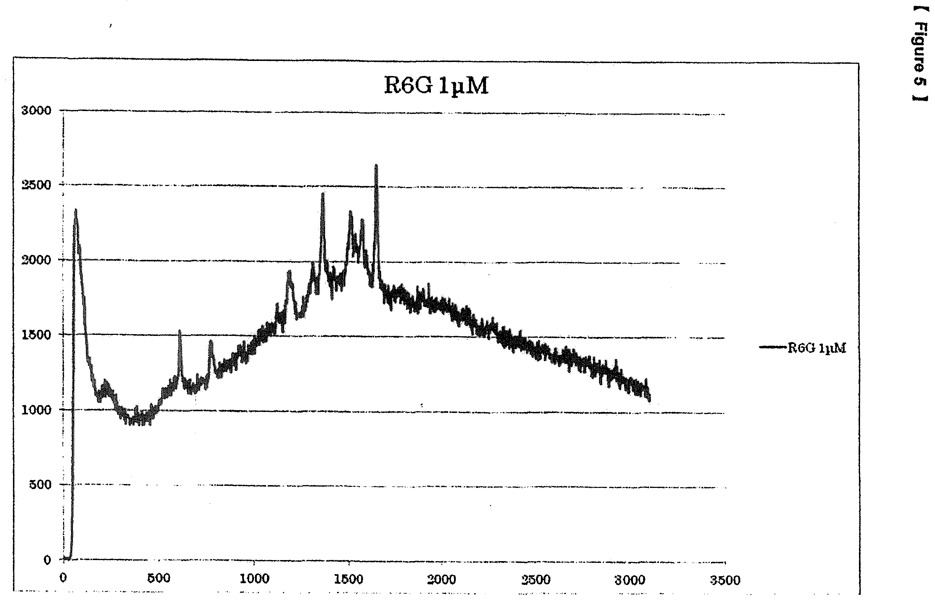

   Fig.5 is a graph showing a Raman scattering spectrum of rhodamine 6G of 1 µM on Ag complex substrate prepared by deposition of Ag thiosulfate aqueous solution on phosphor bronze according to the present invention method.
[Figure 6 (a)]
   Fig.6(a) is a graph showing a Raman scattering spectrum of antiboy on phosphor bronze substrate prepared by antibody added aqueous solution of Ag thiosulfate according to the present invention method.
[Figure 6 (b)]
   Fig.6(b) is a graph showing a Raman scattering spectrum of antigen-antiboy on phosphor bronze substrate prepared by a drop of antigen on the substrate of Fig.6(a).
[Figure 7]
   Fig.7 is a drawing showing a process for preparing a SERS substrate.

### [The embodiments to carry out the invention]

The preferred embodiment of this invention is explained referring to the following drawings in detail.
As shown in Fig.7(a) - (c), a circle dish shaped metal plate having a thickness of about o.1 mm made by a means of punching is adhered on a plate of glass or plastics. The substrate is provided with the circle dish shaped substrate which can receive drops of the aqueous solution and there is a rise of the droplet 3 on the metal substrate as shown in Fig.7 (b). Thereafter, the droplet was blown out by a blow of Nitrogen gase and as a result there is left an agglomeration area 4 formed on the surface of metal substrate, where nano clusters are formed as the quantum dots as shown in Fig.7 (c). In place of the thin metallic plate 2, a metal film may be formed by a chemical plating or a chemical vapor deposition.

### [Example 1]

The aquous solution of plasmon metal complex is prepared by a predetermined amount of silver chloride is dissolved in an aqueous solution of sodium thiosulfate according to a known standard method. The aqueous solution is diluted with pure water up to a concentration of 500 - 2000ppm (Ag weight equivalent), and amino acid (L- alanine) of 10 - 20ppm is added to have a colorless solution containing Ag nano clusters (Ag complex). One droplet (10µL) of the metal complex aqueous solution is put on the brass substrate (Cu 60; Sn 40) and 3 minute later the droplet on the metal substrate was blown off and dried out to have a Surface Plasmon Resonance Exicitation (SERS) substrate. Fig.2 is a photograph of the scanning type electron microscope (20,000 times) of the surface profile of the SERS substrate. From the profile as shown in Fig.2, it is confirmed that a hexagonal plate-like crystal of 100 - 150nm is formed on the substrate. In case of Ag complex crystals formed on the phosphor bronze substrate, the photographs (50,000 and 200,000 times) of the scanning type electron microscope show formation of a lot of nano metal dots encapsulated in the quantum crystals as shown in Fig.3 (a) (b). On the resultant brass substrate prepared by 3 minute agglomeration of the metal complex, Bi-pyridine droplets (1 0µL) of 10 mM, 1µM and 100 nM are put down and the effect of surface plasmon enhancement are measured by using Raman Station 400 made in Parkin Elmer Japan Co. Ltd with a laser having a wavelength of 785 nm (resolution:4.0 cm-1, laser output 300mW, spot size 100µΦ). Raman spectrum up to 100 nM can be confirmed as shown Fig.1. Compared with SERS substrate prepared through CVD by Dr. Vladimir Poponin wherein a Raman scattering spectrum of 100 µM can be detected, the present substrate can get a sensitivity of 1,000 times magnification. It can be thought that 1000 times magnification is caused by the quantum dots made of Ag nano cluster in the hexagonal plate-like crystal.

### [Example 2]

The Ag complex aqueous solution of 1,000 ppm (Ag weight equivalent) is prepared and one droplet of the aqueous solution is put on the phosphor bronze substrate and after 3 minutes the droplet was blown off by Nitrogen gas to stop agglomeration of the metal complex. On each of the substrates, pure water or 4,4'-bipyridine of 100 nM are put and the effect of surface plasmon enhancement was measured by Raman spectroscopy made in Ramuda Vision Co. Ltd. with laser having a wavelength of 785 nm (Output:80mmW, Spot size: 50µΦ). The Raman spectrum can be confirmed up to 100nM, as shown in Fig.4 (a) and (b).

### [Example 3]

In place of 4,4'-bipyridine, Rhodamine 6G (R6G) is used as a tgarget sample to be detected. The aqueous solution of Ag thiosulfate complex is prepared and the phosphor bronze substrate is used. The R6G aqueous solution is put a drop on the metal complex substrate and the Raman spectrum of 1µM can be confirmed by Raman spectroscopy (laser having a wavelength of 514 nm) made by Caiser Co. Ltd as shown in Fig.5. Compared with SERS substrate prepared through CVD by Dr. Vladimir Poponin wherein a Raman scattering spectrum of 100 µM can be detected, the present substrate can get a sensitivity of 1,000 times magnification.

### [Example 4]

### Humanized IgE monoclonal antibody (antibody concentration 1.23mg/ml)

(Mlkuri Immuno Laboratory Co. Ltd. Lot.No.214-01-002 : Solution PBS : including 0.09% sodium azide) was diluted ten times with pure water and is mixed with Ag thiosulfate aqueous solution of 1,000ppm (Ag weight equivalent) without amino acid at a mixture ratio of 1 to 1. The resultant solution was put a drop on the phosphor bronze in a same manner as Example 1 to prepare a measuring substrate for SERS.

After the confirmation that the Raman spectrum of the measuring substrate prepared by blending of the metal complex and the antibody, is obtained by irradiation of the laser of the excitation wavelength 514nm as shown Fig. 6 (a), an antigen was put a drop on the measuring substrate and was subjected to a Raman measuring to obtain the Raman spectrum, which was confirmed as shown Fig.6 (b).

Compared with those Raman spectrum, it is confirmed that we can get a peak appeared near 1350cm -1 due to an antigen-antibody reaction, so that we can detected as a result of the antigan-antibody reaction when both were compared.

### [A comparative example]

In place of Ag complex solution used in Example 1, Ag nano colloid solution of 5,000 ppm (Ag weight equivalent) containing 2-pyroridon of 100 to 150 ppm as dispersant, was used while a substrate was prepared in a same manner as Example 1. As Ag nano clusters was much agglomerated as some solid-like points, there can not be observed a surface plasmon resonance exicitation effect.

### [Possibility of utilization in industry]

Therefore, it is guessed that the present quantum crystals of metal complex made according to the present invention has been firstly produced from the aqueous solution in the world. In case of Au, Ag, Cu or Pt, compared with nano dots produced by physical methods such as Vapor Deposition and so on, the present inventive quantum crystals obtained a thousand times magnification of surface plasmon resonance exicitation effect, so that the quantum crystals are useful for surface plasmon resonance elements such as a SERS deteting substrate, a photoelectric tranducer of solar-cells, a scanning near field optical microscopy element, and a metal needle for a medical thermo-therapy.

In the above examples, although phosphor bronze and brass were used as a substrate metal, any other metal substrate are used corresponding to a kind of the metal of nano cluster. It is preferable that the substrate metal should be selected from a metal having a lower electrode potential than that of nano cluster. In case of silver nano cluster, copper and phosphor bronze substrate can be used in place of bronze. Though the metal carrier is usually in a form of a board-shaped, a particle-shaped, a needle-shaped and a capillary-shaped ones, any other forms can be used depending on the kind of use, wherein the metal complex crystals is deposited on on this surface, and it is desirable to make it form the quantum crystal which encapsulates metal nano cluster.

## Claims

1. A method of producing a quantum crystal of an Ag complex having a plasmon enhancement effect, which comprises:
a step of providing an aqueous solution of the Ag complex,
a step of contacting the aqueous solution of the Ag complex with a carrier of Cu or Cu alloy showing an electrode potential lower than that of Ag or with the Cu or Cu alloy carrier being adjusted at an electrode potential lower than that of Ag in the aqueous solution, and
a step of depositing the Ag complex from the aqueous solution on the Cu or Cu alloy carrier to form and arrange quantum crystals of the Ag complex on the Cu or Cu alloy carrier,
wherein the deposition is taking place due to electro-potential difference between the Ag complex and the Cu or Cu alloy carrier.

2. The method of producing a quantum crystal of the Ag complex having a plasmon enhancement effect according to claim 1, wherein the Ag complex is silver thiosulfate.

3. The method of producing a quantum crystal of the Ag complex having a plasmon enhancement effect according to claim 1, wherein the Cu or Cu alloy carrier is selected from the group consisting of Cu, bronze and phosphor bronze.

4. The method of producing a quantum crystal of the Ag complex having a plasmon enhancement effect according to claim 1, wherein the aqueous solution of the Ag complex is in a concentration of 500 to 5,000 ppm.

5. The method of producing a quantum crystal of the Ag complex having a plasmon enhancement effect according to claim 1, wherein the aqueous solution of the Ag complex is in a concentration of 500 to 2,000 ppm.

6. The method of producing a quantum crystal of the Ag complex having a plasmon enhancement effect according to claim 1, wherein a dispersant is in a range of 1/5 to 1/150 of Ag atomic weight equivalent.

7. The method of producing a quantum crystal of the Ag complex having a plasmon enhancement effect according to claim 1, which further comprises a step of adding antibody into the aqueous solution of the Ag complex and a step of depositing the Ag complex together with the antibody on the Cu or Cu alloy carrier substrate to prepare a measuring substrate for antigen-antibody reaction.

## Patentansprüche

1. Verfahren zur Herstellung eines Quantenkristalls eines Ag-Komplexes mit einem Plasmon-Verstärkungseffekt, umfassend:
einen Schritt zum Bereitstellen einer wässrigen Lösung des Ag-Komplexes,
einen Schritt zum Kontaktieren der wässrigen Lösung des Ag-Komplexes mit einem Träger aus Cu oder Cu-Legierung, der ein Elektrodenpotential aufweist, das niedriger als das von Ag oder mit dem Cu oder Cu-Legierungsträger ist, der auf ein Elektrodenpotential eingestellt ist, das niedriger als das von Ag in der wässrigen Lösung ist, und
einen Schritt zum Ablagern des Ag-Komplexes aus der wässrigen Lösung auf dem Cu oder Cu-Legierungsträger, um Quantenkristalle des Ag-Komplexes auf dem Cu oder Cu-Legierungsträger zu bilden und anzuordnen,
wobei das Ablagern aufgrund der Elektropotentialdifferenz zwischen dem Ag-Komplex und dem Cu oder Cu-Legierungsträger stattfindet.

2. Verfahren zur Herstellung eines Quantenkristalls des Ag-Komplexes mit einem Plasmon-Verstärkungseffekt nach Anspruch 1, wobei der Ag-Komplex Silberthiosulfat ist.

3. Verfahren zur Herstellung eines Quantenkristalls des Ag-Komplexes mit einem Plasmon-Verstärkungseffekt nach Anspruch 1, wobei das Cu oder Cu-Legierungsträger aus der Gruppe, bestehend aus Cu, Bronze und Phosphorbronze, ausgewählt ist.

4. Verfahren zur Herstellung eines Quantenkristalls des Ag-Komplexes mit einem Plasmon-Verstärkungseffekt nach Anspruch 1, wobei die wässrige Lösung des Ag-Komplexes in einer Konzentration von 500 bis 5000 ppm vorliegt.

5. Verfahren zur Herstellung eines Quantenkristalls des Ag-Komplexes mit einem Plasmon-Verstärkungseffekt nach Anspruch 1, wobei die wässrige Lösung des Ag-Komplexes in einer Konzentration von 500 bis 2.000 ppm vorliegt.

6. Verfahren zur Herstellung eines Quantenkristalls des Ag-Komplexes mit einem Plasmon-Verstärkungseffekt nach Anspruch 1, wobei ein Dispergiermittel in einem Bereich von 1/5 bis 1/150 Ag-Atomgewichtsäquivalent liegt.

7. Verfahren zur Herstellung eines Quantenkristalls des Ag-Komplexes mit einem Plasmon-Verstärkungseffekt nach Anspruch 1, das ferner einen Schritt zum Hinzufügen von Antikörper in die wässrige Lösung des Ag-Komplexes und einen Schritt des Ablagerns des Ag-Komplexes zusammen mit dem Antikörper auf dem Cu oder Cu-Legierungsträgersubstrat umfasst, um ein Mess-substrat für die Antigen-Antikörper-Reaktion herzustellen.

## Revendications

1. Un procédé de production d'un cristal quantique d'un complexe Ag ayant un effet d'amélioration du plasmon, qui comprend :
une étape consistant à fournir une solution aqueuse du complexe Ag,
une étape de mise en contact de la solution aqueuse du complexe Ag avec un porteur de Cu ou d'alliage Cu représentant un potentiel d'électrode inférieur à celui d'Ag ou avec le porteur de Cu ou d'alliage Cu étant ajusté à un potentiel d'électrode inférieur à celui d'Ag dans la solution aqueuse, et
une étape de dépôt du complexe Ag à partir de la solution aqueuse sur le porteur de Cu ou d'alliage Cu pour former et organiser des cristaux quantiques du complexe Ag sur le porteur de Cu ou d'alliage Cu,
dans lequel le dépôt a lieu en raison de la différence de potentiel électrique entre le complexe Ag et le porteur de Cu ou d'alliage Cu.

2. Le procédé de production d'un cristal quantique du complexe Ag ayant un effet d'amélioration du plasmon selon la revendication 1, dans lequel le complexe Ag est le thiosulfate d'argent.

3. Le procédé de production d'un cristal quantique du complexe Ag ayant un effet d'amélioration du plasmon selon la revendication 1, dans lequel le porteur de Cu ou d'alliage Cu est choisi dans le groupe composé de Cu, de bronze et de bronze phosphoreux.

4. Le procédé de production d'un cristal quantique du complexe Ag ayant un effet d'amélioration du plasmon selon la revendication 1, dans lequel la solution aqueuse du complexe Ag est dans une concentration de 500 à 5 000 ppm.

5. Le procédé de production d'un cristal quantique du complexe Ag ayant un effet d'amélioration du plasmon selon la revendication 1, dans lequel la solution aqueuse du complexe Ag est dans une concentration de 500 à 2 000 ppm.

6. Le procédé de production d'un cristal quantique du complexe Ag ayant un effet d'amélioration du plasmon selon la revendication 1, dans lequel un dispersant est dans une gamme de 1/5 à 1/150 d'équivalent en poids atomique Ag.

7. Le procédé de production d'un cristal quantique du complexe Ag ayant un effet d'amélioration du plasmon selon la revendication 1, qui comprend en outre une étape d'addition d'anticorps dans la solution aqueuse du complexe Ag et une étape de dépôt du complexe Ag avec l'anticorps sur le substrat de porteur de Cu ou d'alliage Cu pour préparer un substrat de mesure pour la réaction antigène-anticorps.
